# EUROPÄISCHE PATENTSCHRIFT

(11) **EP 3 247 433 B1**
(45) Veröffentlichungstag und Bekanntmachung des Hinweises auf die Patenterteilung: **28.10.2020**
(21) Anmeldenummer: 16701106.3
(22) Anmeldetag: 18.01.2016
(51) Int. Cl.: A61M 11/00, A61M 15/00, B05B 17/06

(54) **VERFAHREN ZUR REINIGUNG EINER SCHWINGUNGSFÄHIGEN MEMBRAN EINES MEMBRANAEROSOLGENERATORS UND KOMBINATION AUS REINIGUNGSVORRICHTUNG UND REINIGUNGSFLÜSSIGKEIT FÜR EINE SOLCHE REINIGUNG**
METHOD FOR CLEANING A MEMBRANE OF A MEMBRANE AEROSOL GENERATOR, WHICH MEMBRANE CAN VIBRATE, AND COMBINATION OF A CLEANING DEVICE AND A CLEANING LIQUID FOR SUCH CLEANING
PROCÉDÉ POUR LE NETTOYAGE D'UNE MEMBRANE OSCILLANTE D'UN GÉNÉRATEUR D'AÉROSOL À MEMBRANE ET COMBINAISON D'UN DISPOSITIF DE NETTOYAGE ET D'UN LIQUIDE DE NETTOYAGE POUR UN TEL NETTOYAGE

(30) Priorität: 23.01.2015 DE 102015201134
(43) Veröffentlichungstag der Anmeldung: 29.11.2017
(73) Patentinhaber: PARI Pharma GmbH, 82319 Starnberg (DE)
(72) Erfinder: MÜLLER, Dominik, 82431 Kochel (DE)
(74) Vertreter: Hoffmann Eitle
(86) Internationale Anmeldenummer: PCT/EP2016/050881
(87) Internationale Veröffentlichungsnummer: WO 2016/116396

(56) Entgegenhaltungen:
- EP-A1- 1 875 936
- WO-A1-96/23679
- WO-A1-03/035153
- WO-A2-2006/108556
- US-A1- 2002 023 639

## Beschreibung

Die vorliegende Erfindung betrifft ein Verfahren zur Reinigung einer schwingungsfähigen Membran eines Membranaerosolgenerators eines Inhalationstherapiegeräts laut Anspruch 1.

Des Weiteren umfasst die vorliegende Erfindung eine Kombination einer Reinigungsvorrichtung und einer Reinigungsflüssigkeit laut Anspruch 8, wobei die Reinigungsvorrichtung für die Reinigung einer schwingungsfähigen Membran eines Membranaerosolgenerators eines Inhalationstherapiegeräts ausgelegt ist.

Mit Inhalationstherapiegeräten werden therapeutisch wirksame oder medikamenthaltige Flüssigkeiten mit Hilfe eines Aerosolerzeugers zu einem Aerosol vernebelt, das aus lungengängigen Partikeln besteht. Das erzeugte Aerosol wird einem Patienten im Rahmen einer Inhalationstherapie zum Einatmen dargeboten, wodurch die therapeutisch wirksame Flüssigkeit oder das Medikament in die Atemwege des Patienten gelangt.

Bei besonders effektiven Inhalationstherapiegeräten wird als Aerosolerzeuger ein Membranaerosolgenerator für die Erzeugung des Aerosols eingesetzt. Die zu vernebelnde Flüssigkeit wird in einem Vorratsbehälter bevorratet und der Membran des Membranaerosolgenerators auf deren einer Seite, der Flüssigkeitsseite, zugeführt. Wird die Membran des Membranaerosolgenerators in Schwingungen versetzt, wird die Flüssigkeit durch Öffnungen der Membran hindurch transportiert und auf der anderen Seite der Membran, der Aerosolseite, in Form eines Aerosols abgegeben. Um die Membran in Schwingungen zu versetzen, umfasst der Membranaerosolgenerator neben der Membran einen Schwingungserzeuger, beispielsweise ein ringförmiges piezo-elektrisches Element und ein ringförmiges Substrat, die miteinander und mit der Membran derart verbunden sind, dass ein dem piezo-elektrischen Element zugeführtes Ansteuerungssignal Schwingungen des Schwingungserzeugers veranlasst, die die Schwingungen der Membran hervorrufen.

Als ein Beispiel eines derartigen Inhalationstherapiegeräts offenbart EP 1 304 131 A1 eine Inhalationstherapievorrichtung mit einer schwingungsfähigen Membran für die Verneblung einer Flüssigkeit mit einer Schwingungserzeugungseinrichtung, die zumindest eine Anschlusseinrichtung für die Zuführung eines Ansteuersignals aufweist, durch das die Membran in Schwingungen versetzt wird, so dass eine auf einer Seite der Membran anstehende Flüssigkeit durch die Membran hindurch vernebelt wird und auf der anderen Seite der Membran als Aerosol vorliegt.

Inhalationstherapiegeräte mit Membranaerosolgenerator zeichnen sich durch eine hohe Effizienz, hohe Dosisgenauigkeit und kurze Therapiezeiten aus, so dass sich Inhalationstherapiegeräte mit Membranaerosolgenerator in besonderem Maße für die Applikation sehr teurer und/oder hochwirksamer Medikamente eignen. Daneben sind Inhalationstherapiegeräte mit Membranaerosolgenerator über einen langen Zeitraum mit zufriedenstellenden Ergebnissen einsetzbar. Es kann nach zahlreichen Therapiesitzungen aus verschiedenen Gründen (abhängig von der Sorgfalt, mit der die vorgeschriebenen Reinigung durchgeführt wird, Umwelteinflüssen, Art der verwendeten Medikamente, etc.) zu einer Verlangsamung der Aerosolerzeugung kommen, was aber in der Regel nicht zu einer Verschlechterung der Aerosolqualität, wie Tröpfchengrößenverteilung (MMD, GSD) führt.

In praktisch allen Anwendungsfällen ist eine übermäßige Verlängerung der Therapiesitzungen nicht hinnehmbar, da dies die Adhärenz zur verordneten Therapie verschlechtern könnte und stets eine schnellstmögliche Verabreichung einer bestimmten Dosis des Medikaments angestrebt wird. Denn keinem Patienten/Anwender des Inhalationstherapiegeräts sollen unnötig lange Therapiezeiten zugemutet werden. Grundsätzlich sind demnach lange Therapiezeiten unerwünscht. Daneben sind die vergleichsweise hohen Herstellungskosten von hochwertigen Inhalationstherapiegeräten mit Membranaerosolgenerator zu beachten. Deshalb besteht das Bedürfnis, die Lebensdauer des Inhalationstherapiegeräts, also den Zeitraum, über den ein Inhalationstherapiegerät mit Membranaerosolgenerator verwendet werden kann, weiter zu verlängern, ohne dass es zu einer deutlichen Verlängerung der Therapiezeiten und anderer therapierelevanten Eigenschaften kommt.

Untersuchungen von in Membranaerosolgeneratoren eingesetzten Membranen mit einem Rasterelektronenmikroskop haben ergeben, dass teilweise trotz vorschriftsmäßiger Reinigung des Membranaerosolgenerators gelegentlich ein zunehmend großer Anteil der Membranöffnungen durch Material verschiedener Herkunft verstopft wird, wodurch die Ausbringrate (Output) der Aerosolerzeugung verringert bzw. die Geschwindigkeit der Aerosolerzeugung verlangsamt wird, was sich negativ auf die Therapiesitzungsdauer auswirkt und diese unvorteilhaft verlängert.

EP 1 875 936 A1 offenbart ein Verfahren und eine Vorrichtung für die Reinigung einer schwingungsfähigen Membran eines Membranaerosolgenerators eines Inhalationstherapiegeräts, wobei die Reinigung der Membran durch einen Rückspülbetrieb der Membran erreicht wird. Die Reinigungsflüssigkeit wird der Membran auf der Aerosolabgabeseite (im üblichen Betrieb) der Membran zugeführt und anschließend werden Membranschwingungen aktiviert, die die Reinigungsflüssigkeit durch die Membran transportieren, so dass diese auf der Flüssigkeitszuführungsseite (im üblichen Betrieb) der Membran abtropft.

Vor dem oben dargelegten Hintergrund liegt der vorliegenden Erfindung die Aufgabe zugrunde, die Effizienz der Reinigung einer schwingungsfähigen Membran eines Membranaerosolgenerators eines Inhalationstherapiegeräts zu erhöhen, so dass der Zeitraum, über den das Inhalationstherapiegerät auf hohem Qualitätsniveau verwendet werden kann, verlängert wird.

Diese Aufgabe wird durch ein Verfahren mit den Merkmalen des Anspruchs 1 und durch eine Kombination einer Reinigungsvorrichtung und einer Reinigungsflüssigkeit mit den Merkmalen des Anspruchs 8 gelöst. Vorteilhafte Ausführungsformen folgen aus den übrigen Ansprüchen.

Gemäß einem ersten Aspekt stellt die vorliegende Erfindung ein Verfahren zur Reinigung einer schwingungsfähigen Membran eines Membranaerosolgenerators eines Inhalationstherapiegeräts bereit, bei dem der Membran eine Reinigungsflüssigkeit zugeführt wird und die Membran derart in Schwingungen versetzt wird, dass die Reinigungsflüssigkeit durch die Öffnungen der Membran gefördert wird. Die Reinigungsflüssigkeit enthält wenigstens ein Tensid.

Die schwingungsfähige Membran kann aus einem Metall, wie z. B. Stahl, insbesondere Edelstahl, Aluminium oder einem anderen elastischen Metall, aus einem Kunststoff oder aus einer Keramik ausgebildet sein.

Durch die Verwendung einer Reinigungsflüssigkeit, die wenigstens ein Tensid enthält, kann die Effizienz der Reinigung der schwingungsfähigen Membran erheblich gesteigert werden.

Die Reinigungsflüssigkeit kann genau ein Tensid oder mehrere unterschiedliche Tenside enthalten. Erfindungsgemäß enthält die Reinigungsflüssigkeit zur Verwendung im erfindungsgemäßen Verfahren wenigstens ein Tensid. Damit eine optimale Reinigungswirkung erzielt wird, beträgt die Konzentration des Tensids oder der Tenside in der Reinigungsflüssigkeit vorzugsweise 0.05 bis 10 Gew.-%, bevorzugter 0.1 bis 5 Gew.-% und besonders bevorzugt 0.5 bis 2 Gew.-%, bezogen auf das Gesamtgewicht der Reinigungsflüssigkeit.

Vorzugweise enthält die Reinigungsflüssigkeit wenigstens ein kationisches Tensid. Die Verwendung von kationischen Tensiden im erfindungsgemäßen Verfahren erwies sich als besonders vorteilhaft, weil dadurch übliche Verunreinigungen an der Membran, wie z.B. proteinhaltige Rückstände, schwerlösliche Medikamentenrückstände sowie Staubpartikel, aus den Öffnungen der Membran besonders effizient entfernt werden können. Zugleich wurde überraschenderweise festgestellt, dass die hydrophilen und aerosolgenerierenden Eigenschaften der Membran und insbesondere deren Durchlässigkeit für pharmazeutische Zubereitungen bzw. Fluide nach dem erfindungsgemäßen Reinigungsverfahren weitgehend unbeeinträchtigt bleiben. Dies ist überraschend, da kationische Tenside bekanntermaßen die Durchlässigkeit von vielen Membranmaterialien negativ beeinflussen und insbesondere zur Reinigung von Membrantextilien ungeeignet sind.

Geeignete kationische Tenside sind typischerweise quartäre Ammoniumsalze, bei denen es sich beispielsweise um Chloride Bromide oder Alkylsulfate handeln kann. Bei kationischen Tensiden in der Reinigungslösung kann es sich z.B. um Tenside wie Di-(C₈₋₂₄)-Alkyl-dimethylammoniumchlorid oder -bromid, vorzugsweise Di-(C₁₂₋₁₈)-Alkyl-dimethylammoniumchlorid oder -bromid, z.B. Distearyl-dimethylammonium-chlorid oder -bromid, Ditalgalkyl-dimethylammoniumchlorid oder -bromid, Dioleyldimethylammoniumchlorid oder -bromid, Dicocosalkyldimethyl-ammoniumchlorid oder -bromid; (C₈₋₂₄)-Alkyl-dimethyl-ethylammoniumchlorid oder -bromid handeln. Ferner bevorzugt sind (C₈₋₂₄)-Alkyl-trimethylammoniumchlorid oder -bromid, vorzugsweise Cetyltrimethyl-ammoniumchlorid oder -bromid und (C₂₀₋₂₂)-Alkyl-trimethylammoniumchlorid oder-bromid, (C₈₋₂₄)- Alkyl-dimethylbenzyl-ammoniumchlorid oder -bromid, vorzugsweise (C₁₂₋₁₈)-Alkyl-dimethylbenzyl-ammoniumchlorid, N-(C₁₀₋₁₈)-Alkyl-pyridiniumchlorid oder-bromid, vorzugsweise N-(C₁₂₋₁₆)-Alkyl-pyridiniumchlorid oder -bromid, N-(C₁₀₋₁₈)-Alkyl-isochinolinium-chlorid,-bromid oder -monoalkylsulfat, N-(C₁₂₋₁₈)-Alkyl-polyoylaminoformylmethyl-pyridiniumchlorid, N-(C₁₂₋₁₈)-Alkyl-N-methyl-morpholinium-chlorid, -bromid oder-monoalkylsulfat, N-(C₁₂₋₁₈)-Alkyl-N-ethyl-morpholinium-chlorid, -bromid oder -monoalkylsulfat; (C₁₆₋₁₈)-Alkyl-pentaoxethyl-ammonium-chlorid oder Diisobutylphenoxyethoxyethyldimethylbenzylammonium-chlorid. Besonders bevorzugt sind unter anderem Benzalkoniumchlorid, Didecyldimethalammoniumchlorid, Dioctyldimethylammoniumchlorid, Cetrimoniumbromid, Cetylpyridiniumchlorid (Hexadecylpyridiniumchlorid), Dimethyldecyloxethylammoniumpropionat, N,N-Didecyl-N-methyl-poly(oxyethyl)ammoniumpropionat und Isotridecanolethoxylat.

Vorzugsweise weist das kationische Tensid in der Reinigungslösung zugleich bakterizide und/oder antifungale und/oder antivirale Eigenschaften auf, so dass die Membran des Membranaerosolgenerators durch das erfindungsgemäße Verfahren auch weitestgehend desinfiziert werden kann. Dies ist insbesondere von Vorteil, wenn das Inhalationstherapiegerät für die Therapie infektiöser Erkrankungen der Lunge eingesetzt wird.

Die Konzentration des kationischen Tensids oder der kationischen Tenside in der Reinigungsflüssigkeit liegt vorzugsweise im Bereich von 0.03 bis 10 Gew.-%, bevorzugter bei 0.07 bis 4 Gew.-%, besonders bevorzugt bei 0.3 bis 2 Gew.-%, bezogen auf das Gesamtgewicht der Reinigungsflüssigkeit.

In einer weiteren Ausführungsform enthält die Reinigungsflüssigkeit wenigstens ein nichtionisches Tensid. Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) oder Propylenoxid (PO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, zum Beispiel aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C₁₂₋₁₄-Alkohole mit 3 EO, 4 EO oder 7 EO, C₉₋₁₁-Alkohol mit 7 EO, C₁₃₋₁₅-Alkohole mit 3 EO, 5 EO, 7 EO oder 8 EO, C₁₂₋₁₆-Alkohole mit 5 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 7 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf. Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO. Auch nichtionische Tenside, die EO- und PO-Gruppen zusammen im Molekül enthalten, sind erfindungsgemäß einsetzbar. Hierbei können Blockcopolymere mit EO-PO-Blockeinheiten bzw. PO-EO-Blockeinheiten eingesetzt werden, aber auch EO-PO-EO-Copolymere bzw. PO-EO-PO-Copolymere. Selbstverständlich sind auch gemischt alkoxylierte Niotenside einsetzbar, in denen EO- und PO-Einheiten nicht blockweise, sondern statistisch verteilt sind. Solche Produkte sind durch gleichzeitige Einwirkung von Ethylen- und Propylenoxid auf Fettalkohole erhältlich.

Als Beispiele für geeignete nichtionische Tenside sind insbesondere EO-Derivate wie PEG-90, PEG-150, PEG-180 oder Ceteareth, wie z.B. Ceteareth-80, zu nennen.

Die Konzentration des nichtionischen Tensids oder der nichtionischen Tenside in der Reinigungsflüssigkeit liegt vorzugsweise im Bereich von 0.01 bis 5 Gew.-%, bevorzugter bei 0.03 bis 3 Gew.-%, besonders bevorzugt bei 0.1 bis 1 Gew.-%, bezogen auf das Gesamtgewicht der Reinigungsflüssigkeit.

In einer Ausführungsform enthält die Reinigungsflüssigkeit wenigstens ein kationisches Tensid in Kombination mit wenigstens einem nichtionischen Tensid. Die Verwendung einer solchen Kombination von Tensiden in der Reinigungsflüssigkeit ermöglicht eine besonders effiziente gleichzeitige Entfernung von Rückständen an der Membran mit unterschiedlichen physikalischen Eigenschaften und somit eine besonders effiziente Reinigung der Membran.

Anstelle von oder zusätzlich zu den kationischen und/oder nichtionischen Tensiden kann die Reinigungsflüssigkeit auch wenigstens ein anionisches Tensid enthalten. Als anionische Tenside werden beispielsweise solche vom Typ der Sulfonate und Sulfate eingesetzt. Als Tenside vom Sulfonat-Typ kommen dabei vorzugsweise C₉₋₁₃-Alkylbenzolsulfonate, Olefinsulfonate, d.h. Gemische aus Alken- und Hydroxyalkansulfonaten sowie Disulfonaten in Betracht. Geeignet sind auch Alkansulfonate. Ebenso sind auch die Ester von α-Sulfofettsäuren (Estersulfonate), zum Beispiel die α-sulfonierten Methylester der hydrierten Kokos-, Palmkern- oder Talgfettsäuren geeignet.

Die Reinigungsflüssigkeit kann ferner wenigstens eine Säure enthalten. Die Reinigungsflüssigkeit kann genau eine Säure oder mehrere unterschiedliche Säuren enthalten.

Indem eine Reinigungsflüssigkeit verwendet wird, die wenigstens eine Säure enthält, können insbesondere Kalkverunreinigungen und durch Kalk entstandene Verstopfungen an der Membran besonders effektiv entfernt werden. Überdies kann die Oberfläche der Membran durch die in der Reinigungsflüssigkeit enthaltene wenigstens eine Säure passiviert werden, um so die Wahrscheinlichkeit zukünftiger Verunreinigungen und Verstopfungen der Membran zu verringern.

Die wenigstens eine Säure kann eine organische Säure, insbesondere Zitronensäure oder Essigsäure, sein.

Die Reinigungsflüssigkeit für das erfindungsgemäße Verfahren weist vorzugsweise einen pH-Wert zwischen 4 und 8, bevorzugter zwischen 5 und 7.5 und besonders bevorzugt zwischen 5.5 und 7 auf. Der gewünschte pH-Wert der Reinigungsflüssigkeit kann beispielsweise dadurch erreicht werden, dass die Reinigungsflüssigkeit wenigstens eine Säure und/oder wenigstens einen Puffer enthält.

Als Säuren für die Reinigungsflüssigkeit können nichttoxische wasserlösliche organische oder anorganische Säuren verwendet werden, mit denen sich der gewünschte pH-Wert der Reinigungsflüssigkeit einstellen lässt. Insbesondere ist die Verwendung von organischen Säuren bevorzugt, da durch deren Pufferwirkung ggf. in Anwesenheit der Salze dieser Säuren der pH-Wert der Reinigungsflüssigkeit während der Reinigung im gewünschten Bereich gehalten werden kann. Dies ist insbesondere dann von Vorteil, wenn die Membran durch basische oder saure Rückstände verunreinigt ist.

Als bevorzugte Säuren zur Verwendung in der Reinigungsflüssigkeit sind insbesondere organische Säuren, wie beispielsweise Ameisensäure, Essigsäure, Zitronensäure, Weinsäure, Apfelsäure oder Sulfamidsäure, zu nennen. Insbesondere ist die Verwendung von Zitronensäure in der Reinigungsflüssigkeit vorteilhaft, da hierdurch eine hervorragende Reinigungswirkung erzielt wird. Des Weiteren weist Zitronensäure sowie deren Salze eine sehr gute Pufferwirkung bei bevorzugten pH-Werten auf.

Um die Pufferwirkung der Reinigungsflüssigkeit zu erhöhen, können dieser zusätzliche Puffer wie beispielsweise Natriumcitrat oder Natriumacetat zugesetzt werden.

Insbesondere kann die Reinigungsflüssigkeit einen oder mehrere Puffer zum Einstellen des pH-Werts der Reinigungsflüssigkeit enthalten. Die Verwendung eines oder mehrerer solcher Puffer ist auch insbesondere bei Reinigungsflüssigkeiten vorteilhaft, die ferner wenigstens ein Enzym enthalten. Mittels des Puffers oder der Puffer kann der pH-Wert der Reinigungsflüssigkeit in dem Bereich eingestellt werden, in dem das Enzym seine maximale Wirkung, z. B. zur Spaltung von organischen Verbindungen, wie z. B. Proteinen und/oder Peptiden, entfaltet. Somit können Verunreinigungen und Verstopfungen der Membran besonders effektiv entfernt werden.

Die Reinigungsflüssigkeit enthält ferner wenigstens ein Enzym. Die Reinigungsflüssigkeit kann genau ein Enzym oder mehrere unterschiedliche Enzyme enthalten.

Durch die Verwendung einer Reinigungsflüssigkeit, die wenigstens ein Enzym enthält, können insbesondere organische Verunreinigungen und Verstopfungen der Membran besonders effektiv entfernt werden. In dieser Weise kann die Effizienz der Reinigung der Membran weiter erhöht werden.

Das wenigstens eine Enzym ist ein Enzym zur Spaltung von Proteinen und/oder Peptiden.
Für die Verwendung in der Reinigungsflüssigkeit besonders bevorzugte Enzyme sind insbesondere solche aus den Klassen der Hydrolasen wie der Proteasen, (Poly)Esterasen, Lipasen bzw. lipolytisch wirkende Enzyme, Amylasen, Cellulasen bzw. andere Glykosylhydrolasen, Hemicellulase, Cutinasen, β-Glucanasen, Oxidasen, Peroxidasen, Mannanasen, Perhydrolasen, Oxireduktasen und/oder Laccasen. Um eine besonders effiziente Entfernung der proteinhaltigen und lipidhaltigen Rückstände an der Membran zu gewährleisten, erwies sich die Verwendung mindestens einer Protease und/oder Lipase als besonders vorteilhaft. Überraschenderweise bewirkt die Verwendung der Enzyme im erfindungsgemäßen Verfahren eine hervorragende Reinigungswirkung, ohne dass es zu unerwünschten, durch die Enzyme bedingten Ablagerungen an der Membran kommt.

Vorzugsweise werden Proteasen vom Subtilisin-Typ und insbesondere Proteasen, die aus Bacillus lentus gewonnen werden, eingesetzt. Dabei sind Enzymmischungen, beispielsweise aus Protease und Amylase oder Protease und Lipase bzw. lipolytisch wirkenden Enzymen oder Protease und Cellulase oder aus Cellulase und Lipase bzw. lipolytisch wirkenden Enzymen oder aus Protease, Amylase und Lipase bzw. lipolytisch wirkenden Enzymen oder Protease, Lipase bzw. lipolytisch wirkenden Enzymen und Cellulase, insbesondere jedoch Protease und/oder Lipasehaltige Mischungen bzw. Mischungen mit lipolytisch wirkenden Enzymen von besonderem Interesse. Beispiele für derartige lipolytisch wirkende Enzyme sind die bekannten Cutinasen. Zu den geeigneten Amylasen zählen insbesondere α-Amylasen, Iso-Amylasen, Pullulanasen und Pektinasen.

Die Menge an Enzym bzw. Enzymen in der Reinigungsflüssigkeit beträgt bezogen auf die gesamte Reinigungsflüssigkeit vorzugsweise 0.01 bis 10 Gew.-% und bevorzugter 0.12 bis 3 Gew.-%. Die Enzyme werden bevorzugt als Enzymflüssigformulierung(en) eingesetzt.

Die Reinigungsflüssigkeit kann ferner wenigstens ein Lösungsmittel, insbesondere Alkohol, enthalten. Die Reinigungsflüssigkeit kann genau ein Lösungsmittel oder mehrere unterschiedliche Lösungsmittel enthalten.

Um die Entfernung von Medikamentenrückständen von der Membran zusätzlich zu begünstigen, ist es bevorzugt, dass die Reinigungsflüssigkeit wenigstens ein wasserlösliches organisches Lösungsmittel enthält. Als organische Lösungsmittel hierfür sind insbesondere Alkohole, wie z.B. Ethanol, Isopropanol, Ethylenglykol, Propylenglykol, Glycerin oder deren Gemische, gut geeignet. Insbesondere wird die Verwendung von Ethanol im Hinblick auf seine sehr gute reinigende Wirkung, bakteriziden Eigenschaften und geringe Toxizität besonders bevorzugt. Da die oben genannten organischen Lösungsmittel mit Wasser unbegrenzt mischbar sind, ist deren Gehalt in der Reinigungsflüssigkeit nicht beschränkt und kann an die jeweilige Verunreinigung der Membran angepasst werden.

Vorzugsweise enthält die Reinigungsflüssigkeit bezogen auf die gesamte Reinigungsflüssigkeit nicht mehr als 50 Gew.-%, bevorzugter nicht mehr als 30 Gew.-% und besonders bevorzugt nicht mehr als 10 Gew.-% von einem oder mehreren Lösungsmitteln, insbesondere organischen Lösungsmitteln.

Die Reinigungsflüssigkeit kann ferner wenigstens ein Desinfektionsmittel enthalten. Die Reinigungsflüssigkeit kann genau ein Desinfektionsmittel oder mehrere unterschiedliche Desinfektionsmittel enthalten.

Neben quartiären Ammoniumsalzen und organischen Lösungsmitteln kann die Reinigungslösung weitere Desinfektionsmittel mit anti-mikrobieller und antifungaler Wirkung enthalten. Insbesondere Clorofen, Biphenyl oder Chlorocresol sind zur Verwendung in der Reinigungslösung gut geeignet. Die Verwendung von wenigstens einem zusätzlichen Desinfektionsmittel ist insbesondere bevorzugt, wenn das Inhalationsgerät in einer medizinischen Umgebung von mehreren Patienten verwendet wird.

Vorzugsweise enthält die Reinigungslösung wenigstens ein Bleichmittel, wobei Bleichmittel auf Perborat-Basis, Percarbonat-Basis oder auf Persulfat-Basis besonders bevorzugt sind. Als solche sind insbesondere Natriumperborat, Kaliumperoxodisulfat und/oder Kaliumhydrogenperoxomonosulfat sowie Natriumpercarbonat gut geeignet.

Um eine zusätzliche Verbesserung der Wirkung der Bleichmittel in der Reinigungslösung, insbesondere bei Temperaturen unterhalb 40 °C zu erzielen, kann die Reinigungslösung ferner einen oder mehrere Bleichmittelaktivatoren, wie beispielsweise Tetraacetylethylendiamin (TAED), enthalten.

Des Weiteren können in der Reinigungslösung Duftstoffe, wie beispielsweise Zimtöl, Thymianöl, Teebaumöl, Mandelöl, Calendula, Kamillen-, Pfefferminz- oder Eukalytusextrakte, Menthol, Amylcinnamal, Butylphenyl Methypropional, Citronellol, Hexylcinnamal oder Limonen, enthalten sein, um dem Inhalationstherapiegerät nach dem Reinigungsverfahren einen angenehmen Duft zu verleihen.

Das erfindungsgemäße Verfahren zur Reinigung einer schwingungsfähigen Membran kann mehrere aufeinander folgende Schritte bzw. Durchgänge des Zuführens einer Reinigungsflüssigkeit zu der Membran und des Versetzens der Membran in Schwingungen derart, dass die Reinigungsflüssigkeit durch die Öffnungen der Membran gefördert wird, beinhalten. Insbesondere können zwei oder mehr, drei oder mehr oder vier oder mehr solche Schritte bzw. Durchgänge durchgeführt werden.

Gemäß einer Ausführungsform der Erfindung wird bei dem Verfahren zur Reinigung der schwingungsfähigen Membran die Reinigungsflüssigkeit der Membran auf der Aerosolseite zugeführt und die Membran derart in Schwingungen versetzt, dass die Reinigungsflüssigkeit durch die Öffnungen der Membran auf die Flüssigkeitsseite der Membran gefördert wird.

Hierbei bezeichnet der Begriff "Flüssigkeitsseite" die Seite der Membran, zu der im regulären Verneblerbetrieb, also bei der Verwendung des Inhalationstherapiegeräts für eine Inhalationstherapie, die zu vernebelnde Flüssigkeit zugeführt wird bzw. an der im regulären Verneblerbetrieb die zu vernebelnde Flüssigkeit ansteht. Der Begriff "Aerosolseite" bezeichnet die Seite der Membran, an der die vernebelte Flüssigkeit, also das durch die Schwingungen der Membran erzeugte Aerosol, austritt.

In diesem Fall durchströmt die Reinigungsflüssigkeit die Membran in einer im Vergleich zur Aerosolerzeugung im regulären Verneblerbetrieb entgegengesetzten Richtung. Durch einen solchen Rückspülbetrieb der Membran können Verunreinigungen und Verstopfungen der Membran besonders effektiv entfernt werden.

Die Reinigungsflüssigkeit kann ferner wenigstens einen Hilfsstoff, insbesondere einen Puffer, enthalten, wie oben ausführlich erörtert wurde. Die Reinigungsflüssigkeit kann genau einen Hilfsstoff, insbesondere genau einen Puffer, oder mehrere unterschiedliche Hilfsstoffe, insbesondere mehrere unterschiedliche Puffer, enthalten.

Die Reinigungsflüssigkeit kann ferner wenigstens ein Wasserenthärtungsmittel enthalten. Die Reinigungsflüssigkeit kann genau ein Wasserenthärtungsmittel oder mehrere unterschiedliche Wasserenthärtungsmittel enthalten.

Durch die Verwendung einer Reinigungsflüssigkeit, die ferner wenigstens ein Desinfektionsmittel und/oder wenigstens ein Wasserenthärtungsmittel und/oder wenigstens ein Lösungsmittel enthält, kann die Effizienz der Reinigung der schwingungsfähigen Membran weiter gesteigert werden.

Bei einer Ausführungsform des erfindungsgemäßen Verfahrens wird die Membran auf die Art in Schwingungen versetzt, in der die Membran im Verneblerbetrieb, also bei der Verwendung des Inhalationstherapiegeräts für eine Inhalationstherapie, in Schwingungen versetzt wird. Die Schwingungen der Membran bei deren Reinigung können die gleiche Frequenz und/oder Amplitude wie die Schwingungen der Membran während des Verneblerbetriebs aufweisen. Dieser Ansatz ermöglicht eine besonders einfache Durchführung des erfindungsgemäßen Reinigungsverfahrens.

Das erfindungsgemäße Reinigungsverfahren kann ferner einen Schritt des Einlegens der Membran in ein Ultraschallbad enthalten. Dieser Schritt kann vor oder nach dem Zuführen der Reinigungsflüssigkeit zu der Membran und dem Versetzen der Membran in Schwingungen erfolgen.

Für das Ultraschallbad kann die gleiche Reinigungsflüssigkeit verwendet werden, die der Membran vor dem Versetzen der Membran in Schwingungen zugeführt wird, oder eine unterschiedliche Reinigungsflüssigkeit.

Gemäß einem weiteren Aspekt stellt die vorliegende Erfindung eine Kombination einer Reinigungsvorrichtung und einer Reinigungsflüssigkeit bereit, wobei die Reinigungsvorrichtung für die Reinigung einer schwingungsfähigen Membran eines Membranaerosolgenerators eines Inhalationstherapiegeräts ausgelegt ist. Die Reinigungsvorrichtung umfasst eine Flüssigkeitszuführungseinrichtung, die für die Zuführung der Reinigungsflüssigkeit zu der Membran ausgelegt ist, und eine Schwingungsaktivierungseinrichtung, die für die Aktivierung von Membranschwingungen, so dass die Reinigungsflüssigkeit durch die Öffnungen der Membran gefördert wird, ausgelegt ist. Die Reinigungsflüssigkeit enthält wenigstens ein Tensid und wenigstens ein Enzym. Das wenigstens eine Enzym ist ein Enzym zur Spaltung von Proteinen und/oder Peptiden.

Die Kombination kann die Reinigungsflüssigkeit in flüssiger Form oder als wasserlöslichen Stoff, insbesondere Feststoff, wie z. B. in Form einer oder mehrerer wasserlöslicher Tabletten, beinhalten. In letzterem Fall wird der wasserlösliche Stoff in Wasser gelöst und die so entstehende Lösung der Membran zur Reinigung zugeführt.

Die erfindungsgemäße Kombination der Reinigungsvorrichtung und der Reinigungsflüssigkeit ist für die Durchführung des erfindungsgemäßen Verfahrens zur Reinigung einer schwingungsfähigen Membran eines Membranaerosolgenerators eines Inhalationstherapiegeräts ausgelegt. Folglich bietet die erfindungsgemäße Kombination die bereits oben für das erfindungsgemäße Verfahren dargelegten Vorteile.

Überdies können die oben für das erfindungsgemäße Verfahren beschriebenen Merkmale auch bei der erfindungsgemäßen Kombination verwendet werden. Insbesondere kann die oben für die Verwendung in dem erfindungsgemäßen Verfahren beschriebene Reinigungsflüssigkeit auch für die erfindungsgemäße Kombination verwendet werden.

Die Schwingungsaktivierungseinrichtung kann so ausgelegt sein, dass sie die Membran auf die Art in Schwingungen versetzt, in der die Membran im Verneblerbetrieb in Schwingungen versetzt wird.
Die Flüssigkeitszuführungseinrichtung kann für die Zuführung der Reinigungsflüssigkeit auf die Aerosolseite der Membran ausgelegt sein. Die Schwingungsaktivierungseinrichtung kann für die Aktivierung von Membranschwingungen, so dass die Reinigungsflüssigkeit durch die Öffnungen der Membran auf die Flüssigkeitsseite der Membran gefördert wird, ausgelegt sein.

Bei einer Ausführungsform der Kombination ist die Flüssigkeitszuführungseinrichtung ein Hohlkörper oder im Besonderen ein Hohlzylinder, der mit einer Stirnseite an der Membran oder dem Membranaerosolgenerator derart angeordnet ist, dass eine in den Hohlzylinder eingefüllte Reinigungsflüssigkeit an der Membran ansteht.

Der Hohlzylinder kann als längliches Rohr ausgebildet sein, das für das Einsetzen in ein Mundstück oder in eine Mischkammer des Inhalationstherapiegeräts ausgelegt ist.

Das längliche Rohr kann einen Anschlag aufweisen, der das Einführen des Rohres in das Mundstück eines Inhalationstherapiegeräts begrenzt.

Das längliche Rohr kann zu dem für die Anordnung an der Membran vorgesehenen Ende konisch zulaufen.

Bei einer Ausführungsform der erfindungsgemäßen Kombination ist die Flüssigkeitszuführungseinrichtung trichterförmig und mit einer Öffnung an der Membran oder dem Membranaerosolgenerator derart angeordnet, dass eine in die trichterförmige Flüssigkeitszuführungseinrichtung eingefüllte Reinigungsflüssigkeit an der Membran ansteht.

An der Flüssigkeitszuführungseinrichtung kann eine Dichtung vorgesehen sein, die ein Austreten der Reinigungsflüssigkeit an der Membran bzw. an dem Membranaerosolgenerator verhindert.

Ein Auffangbehälter für das Auffangen der aus der Membran austretenden Reinigungsflüssigkeit, insbesondere der auf der Flüssigkeitsseite der Membran austretenden Reinigungsflüssigkeit, kann vorgesehen sein.

An dem Auffangbehälter kann eine Aufnahme für die Anordnung des Membranaerosolgenerators vorgesehen sein.

Die Flüssigkeitszuführungseinrichtung und der Auffangbehälter können zueinander um eine Achse schwenkbar ausgebildet sein.

Die Flüssigkeitszuführungseinrichtung kann einstückig mit oder ohne einem Deckel ausgebildet sein. Der Deckel kann dabei über ein Scharnier, insbesondere ein Filmscharnier, mit dem Gehäuse der Flüssigkeitszuführungseinrichtung verbunden sein. Die Flüssigkeitszuführungseinrichtung kann aus zwei Hälften bestehen, in welche die Membran eingebracht werden kann, welche durch ein Scharnier, insbesondere ein Filmscharnier, verbunden sind.

In einer weiteren Ausgestaltung kann die Flüssigkeitszuführungseinrichtung zweistückig mit oder ohne einem Deckel ausgebildet sein. Die beiden Hälften der Flüssigkeitszuführungseinrichtung, in welche die Membran eingebracht werden kann, können durch ein Scharnier getrennt werden.

Die Schwingungsaktivierungseinrichtung kann ein Ansteuergerät sein, das ein vorzugsweise elektrisches Ansteuersignal für die Ansteuerung des Membranaerosolgenerators abgibt.

Das Ansteuergerät kann das Steuergerät des Inhalationstherapiegeräts sein.

Die Schwingungsaktivierungseinrichtung, wie z. B. das Ansteuergerät, insbesondere das Steuergerät des Inhalationstherapiegeräts, kann mit dem Membranaerosolgenerator über ein Kabel verbunden sein. Die Schwingungsaktivierungseinrichtung kann ein vorzugsweise elektrisches Ansteuersignal über das Kabel an den Membranaerosolgenerator abgeben. Das Kabel ermöglicht ein Einsetzen des Membranaerosolgenerators in die Reinigungsvorrichtung in einer relativ zu der Ausrichtung bzw. Stellung im regulären Verneblerbetrieb um 180° gedrehten Ausrichtung bzw. Stellung. In diesem Fall wird in einfacher Weise ein Rückspülbetrieb ermöglicht, bei dem die Reinigungsflüssigkeit die Membran in einer im Vergleich zur Aerosolerzeugung im regulären Verneblerbetrieb entgegengesetzten Richtung durchläuft.

Bei einer Ausführungsform der Erfindung kann eine Verbindung zwischen der Schwingungsaktivierungseinrichtung, wie z. B. dem Ansteuergerät, insbesondere dem Steuergerät des Inhalationstherapiegeräts, mit dem Membranaerosolgenerator kabellos erfolgen. Insbesondere kann die Schwingungsaktivierungseinrichtung über eine Steckverbindung mit dem Membranaerosolgenerator verbunden werden. In diesem Fall kann durch die Schwingungsaktivierungseinrichtung ein vorzugsweise elektrisches Ansteuersignal über die Steckverbindung, insbesondere über einen in der Steckverbindung enthaltenen Stecker, an den Membranaerosolgenerator abgegeben werden. Vorzugsweise ist diese Steckverbindung so eingerichtet, dass ein Einsetzen des Membranaerosolgenerators in die Reinigungsvorrichtung, insbesondere in die Schwingungsaktivierungseinrichtung der Reinigungsvorrichtung, wie z. B. das Steuergerät des Inhalationstherapiegeräts, in einer relativ zu der Ausrichtung bzw. Stellung im regulären Verneblerbetrieb um 180° gedrehten Ausrichtung bzw. Stellung ermöglicht wird, so dass ein Rückspülbetrieb in einfacher Weise durchgeführt werden kann. Insbesondere kann eine wie in der europäischen Patentanmeldung EP 14 156 519.2 beschriebene Reinigungsvorrichtung verwendet werden, wobei die Offenbarung der EP 14 156 519.2 hiermit hierin durch Bezugnahme in ihrer Gesamtheit aufgenommen wird. Die Steckverbindung zwischen der Schwingungsaktivierungseinrichtung und dem Membranaerosolgenerator kann in der in der EP 14 156 519.2 beschriebenen und insbesondere in den Figuren 5 bis 8 dieser Patentanmeldung gezeigten Weise ausgebildet sein.

Ein solches Verbinden der Schwingungsaktivierungseinrichtung, wie z. B. des Ansteuergeräts, insbesondere des Steuergeräts des Inhalationstherapiegeräts, mit dem Membranaerosolgenerator in kabelloser Weise, insbesondere durch eine Steckverbindung, ermöglicht eine besonders einfache und zuverlässige Durchführung des Reinigungsverfahrens.
Das Ansteuersignal der Schwingungsaktivierungseinrichtung kann dem Ansteuersignal entsprechen, das während des Verneblerbetriebs, also während der Verwendung des Inhalationstherapiegeräts für eine Inhalationstherapie, dem Membranaerosolgenerator zugeführt wird.

Das Ansteuersignal der Schwingungsaktivierungseinrichtung kann sich von dem Ansteuersignal des Verneblerbetriebs, insbesondere hinsichtlich Amplitude und/oder Frequenz und/oder Frequenzwechsel und/oder Dauer und/oder Sequenz der Aktivierungszeiträume unterscheiden.

Nachfolgend wird die vorliegende Erfindung rein beispielhaft anhand der beigefügten Figuren beschrieben, wobei
- Fig. 1: ein erstes Ausführungsbeispiel einer Reinigungsvorrichtung gemäß der Erfindung zeigt, die für die Reinigung einer schwingungsfähigen Membran eines Inhalationstherapiegeräts ausgelegt ist;
- Fig. 2: ein zweites Ausführungsbeispiel einer erfindungsgemäßen Reinigungsvorrichtung zeigt;
- Fig. 3: ein drittes Ausführungsbeispiel einer erfindungsgemäßen Reinigungsvorrichtung zeigt;
- Fig. 4A: ein Inhalationstherapiegerät mit einem Membranaerosolgenerator zeigt;
- Fig. 4B: ein viertes Ausführungsbeispiel einer erfindungsgemäßen Reinigungsvorrichtung zeigt;
- Fig. 5: experimentelle Daten für die Vernebelungsraten von Membranen für unterschiedliche Reinigungsverfahren zeigt;
- Fig. 6: experimentelle Daten für die Vernebelungsraten von Membranen für eine Reinigung mit deionisiertem Wasser zeigt;
- Fig. 7: experimentelle Daten für die Vernebelungsraten von Membranen für eine Reinigung mit einer Corega-Tabs®-Lösung zeigt;
- Fig. 8: ein Diagramm zeigt, in dem die Effizienz der Reinigung mit deionisiertem Wasser und die Effizienz der Reinigung mit einer Corega-Tabs®-Lösung miteinander verglichen werden; und
- Fig. 9: experimentelle Daten für die Vernebelungsraten von Membranen für eine Reinigung mit Bomix® Plus und eine Reinigung mit einer Corega-Tabs®-Lösung zeigt.

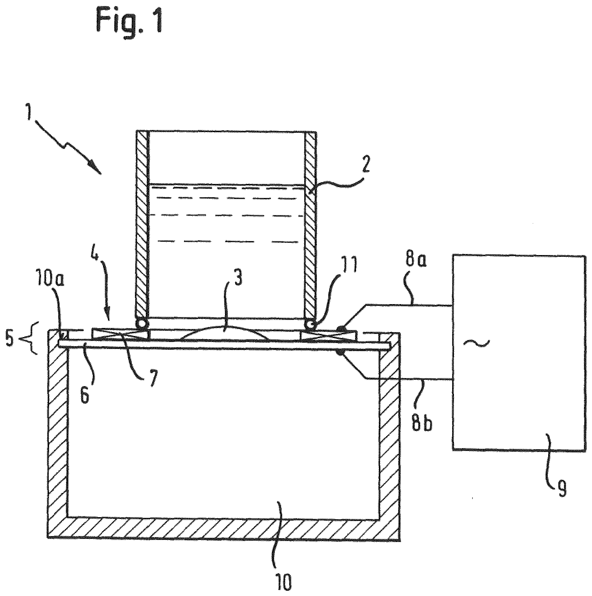

Figur 1 zeigt ein erstes vorteilhaftes Ausführungsbeispiel einer Reinigungsvorrichtung, die für die Reinigung einer schwingungsfähigen Membran eines Membranaerosolgenerators eines Inhalationstherapiegeräts ausgelegt ist und in der erfindungsgemäßen Kombination verwendet werden kann.

Die gezeigte Reinigungsvorrichtung 1 umfasst eine Flüssigkeitszuführungseinrichtung 2 für die Zuführung einer Reinigungsflüssigkeit zu einer Membran 3. Die Zuführung der Reinigungsflüssigkeit erfolgt auf der Seite der Membran 3, auf der sonst während des Vernebelns einer Flüssigkeit das von dem Membranaerosolgenerator erzeugte Aerosol abgegeben wird, also auf der Aerosolseite der Membran.

Der Membranaerosolgenerator 4 umfasst neben der schwingungsfähigen Membran 3 einen Schwingungserzeuger 5, der in dem gezeigten Ausführungsbeispiel aus einem ringförmigen Substrat 6 und einem damit verbundenen ringförmigen piezo-elektrischen Element 7 besteht. Bei dem gezeigten Ausführungsbeispiel besitzt der Schwingungserzeuger 5 damit eine rotationssymmetrische Grundstruktur, deren Rotationsachse in der Zeichnungsebene der Figur 1 liegt.

Wie Figur 1 ferner zeigt, sind Anschlussleitungen 8a, 8b einerseits mit dem Schwingungserzeuger 5 und andererseits mit einer Schwingungsaktivierungseinrichtung 9 für die Aktivierung von Membranschwingungen verbunden.

Gemäß dem in Figur 1 gezeigten Ausführungsbeispiel führt die Schwingungsaktivierungseinrichtung 9 dem Schwingungserzeuger 5 des Membranaerosolgenerators 4 ein elektrisches Ansteuerungssignal zu. Wenn das Ansteuerungssignal zugeführt wird, wird die schwingungsfähige Membran 3 durch den Schwingungserzeuger 5 in Schwingungen versetzt und die Reinigungsflüssigkeit von der Aerosolseite der Membran 3 durch die Membran 3 zur Flüssigkeitsseite der Membran 3 gefördert.

Vorteilhafterweise wird, wie in Figur 1 gezeigt ist, die durch die Öffnungen der Membran geförderte Reinigungsflüssigkeit in einem Auffangbehälter 10 aufgefangen, an dem der Membranaerosolgenerator 4 geeignet angeordnet bzw. angebracht ist. Beispielsweise ist gemäß Figur 1 das ringförmige Substrat 6 an seinem äußeren Umfang in einer Nut 10a des Auffangbehälters 10 fixiert.

Bei dem in Figur 1 gezeigten Ausführungsbeispiel ist die Flüssigkeitszuführungseinrichtung 2 in Form eines an beiden Stirnseiten offenen Hohlzylinders realisiert. Die eine Stirnseite des Hohlzylinders 2 ist so positioniert, dass diese Stirnseite von der Membran 3 bzw. dem Membranaerosolgenerator 4 dicht abgeschlossen wird. An der der Membran zugewandten Stirnseite ist dazu vorteilhaft eine ringförmige Dichtung 11 angeordnet, die an der Membran 3 bzw. dem Membranaerosolgenerator 4 derart anliegt, dass eine in den Hohlzylinder eingefüllte Reinigungsflüssigkeit nicht an der der Membran zugewandten Stirnseite des Hohlzylinders austritt, sondern an der Membran 3 ansteht. Sofern der Hohlzylinder ein ausreichendes Eigengewicht aufweist, um die für die Zuführung der Reinigungsflüssigkeit zu erzielende Dichtigkeit (mit oder ohne Dichtung 11) zu erreichen, sind weitere Maßnahmen zur Fixierung der Flüssigkeitszuführungseinrichtung 2 nicht erforderlich. Andernfalls (oder zusätzlich) können aber Fixierungen für die Flüssigkeitszuführungseinrichtung 2 vorgesehen werden, die die Flüssigkeitszuführungseinrichtung 2 zuverlässig an der für die Zuführung der Reinigungsflüssigkeit geeigneten Position halten.

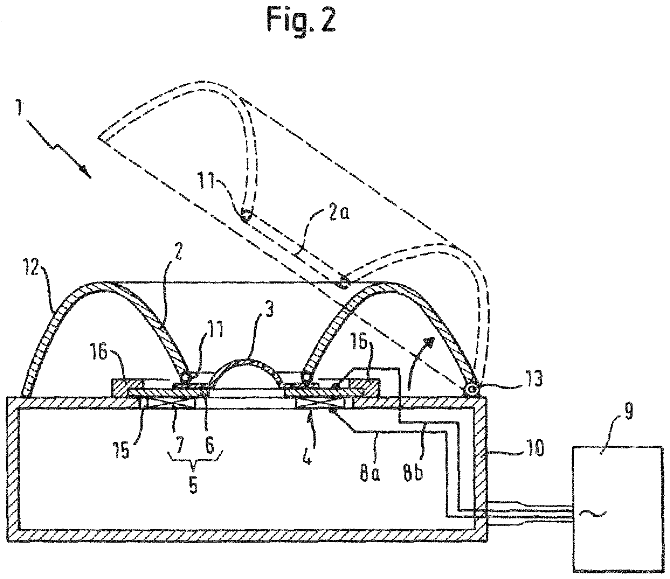

Figur 2 zeigt eine zweite vorteilhafte Ausführungsform einer Reinigungsvorrichtung, die in der erfindungsgemäßen Kombination verwendet werden kann. Bei der in Figur 2 gezeigten Reinigungsvorrichtung 1 ist die Flüssigkeitszuführungseinrichtung 2 für die Zuführung einer Reinigungsflüssigkeit zur Aerosolseite der Membran 3 trichterförmig ausgestaltet. Ferner ist die trichterförmige Flüssigkeitszuführungseinrichtung 2 einstückig mit einem Deckel 12 ausgebildet, der um eine Achse 13 schwenkbar an dem Auffangbehälter 10 befestigt ist. Im geschlossenen Zustand ist die trichterförmige Flüssigkeitszuführungseinrichtung 2 so positioniert, dass das freie offene Ende 2a an der Membran 3 oder dem Membranaerosolgenerator 4 anliegt, so dass eine in die trichterförmige Flüssigkeitszuführungseinrichtung 2 eingefüllte Reinigungsflüssigkeit an der Membran 3 ansteht. Vorteilhafterweise ist an dem freien offenen Ende der Flüssigkeitszuführungseinrichtung 2 eine Dichtung 11 vorgesehen, um ein unerwünschtes Austreten von Reinigungsflüssigkeit aus dem durch die Flüssigkeitszuführungseinrichtung 2 und die Membran 3 begrenzten Volumen zu verhindern.

Für das Entnehmen und das Einsetzen des Membranaerosolgenerators 4 in die Reinigungsvorrichtung 1 gemäß dem zweiten Ausführungsbeispiel kann die Flüssigkeitszuführungseinrichtung 2 zusammen mit dem Deckel 12 um die Achse 13 geschwenkt werden. Der Membranaerosolgenerator 4 ist an einer an dem Auffangbehälter vorgesehenen Aufnahme 15, beispielsweise einer angepassten Öffnung in der dem Deckel zugewandten Oberfläche des Auffangbehälters 10, durch eine oder mehrere Halteeinrichtungen 16, die beispielsweise den Membranaerosolgenerator 4 einklemmen, lösbar fixiert.

Wie in dem ersten Ausführungsbeispiel erfolgt auch in dem zweiten Ausführungsbeispiel die Zuführung der Reinigungsflüssigkeit auf der Aerosolseite der Membran 3, also auf der Seite, auf der sonst während des Vernebelns einer Flüssigkeit das von dem Membranaerosolgenerator erzeugte Aerosol abgegeben wird. Wird der Membranaerosolgenerator 4 angesteuert, so dass die Membran 3 in Schwingungen versetzt wird, tritt die Reinigungsflüssigkeit auf der anderen Seite der Membran 3 aus, also auf der Seite, auf der sonst während eines Verneblerbetriebs die zu vernebelnde Flüssigkeit ansteht (Flüssigkeitsseite).

Dem Membranaerosolgenerator 4 wird, wie bei dem ersten Ausführungsbeispiel, ein Ansteuersignal über Anschlussleitungen 8a und 8b von einer Schwingungsaktivierungseinrichtung 9 zugeführt.

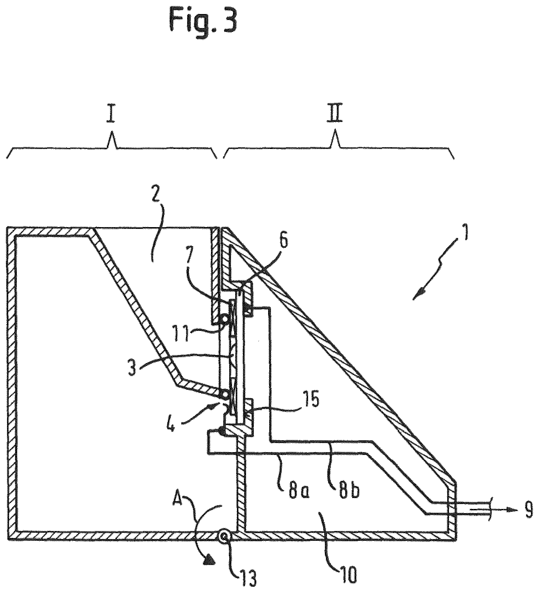

Figur 3 zeigt ein drittes Ausführungsbeispiel einer Reinigungsvorrichtung 1, die in der erfindungsgemäßen Kombination verwendet werden kann. Bei diesem Ausführungsbeispiel ist der Membranaerosolgenerator 4 nicht horizontal, sondern vertikal in der Reinigungsvorrichtung 1 angeordnet, um zu veranschaulichen, dass das erfindungsgemäße Verfahren auch durchführbar ist, wenn die Membran 3 des Membranaerosolgenerators 4 nicht horizontal angeordnet ist. Letztlich kann die Membran 3 des Membranaerosolgenerators 4 beliebig angeordnet werden, solange mit Hilfe der Flüssigkeitszuführungseinrichtung 2 die Reinigungsflüssigkeit der Membran 3 des Membranaerosolgenerators 4, insbesondere der Aerosolseite der Membran 3, zugeführt und die Membran 3 derart in Schwingungen versetzt wird, dass die Reinigungsflüssigkeit durch die Öffnungen der Membran 3 gefördert wird.

Bei dem in Figur 3 gezeigten dritten Ausführungsbeispiel ist die Flüssigkeitszuführungseinrichtung 2 ein teilweise abgeschrägter Behälter, der eine vertikale Öffnung aufweist, die so angeordnet ist, dass die Reinigungsflüssigkeit an der Aerosolseite der Membran 3 ansteht. An der Öffnung der Flüssigkeitszuführungseinrichtung 2 ist vorzugsweise eine Dichtung 11 vorgesehen, die ein unerwünschtes Austreten der Reinigungsflüssigkeit an der Kontaktstelle zwischen Trichter/Dichtung und Membran/Membranaerosolgenerator verhindert.

Wie der in Figur 3 dargestellte Pfeil A andeutet, kann der Teil I der Reinigungsvorrichtung 1, in dem die Flüssigkeitszuführungseinrichtung 2 ausgebildet ist, um eine Drehachse geschwenkt werden, wodurch die Aufnahme 15 für den Membranaerosolgenerator 4 freigegeben wird, die in einem anderen Teil II der Reinigungsvorrichtung 1 ausgebildet ist. In die Aufnahme 15 wird der Membranaerosolgenerator 4 eingelegt und dabei mit Anschlussleitungen 8a und 8b verbunden, über die ein Ansteuerungssignal dem Membranaerosolgenerator 4 zugeführt wird. Wenn der erste Gehäuseteil I der Reinigungsvorrichtung 1 in die in Figur 3 gezeigte Position geschwenkt ist, wird der Membranaerosolgenerator 4 bzw. die Membran 3 in der Aufnahme fixiert, da die Dichtung 11 auf den Membranaerosolgenerator 4 mechanisch einwirkt. Der zweite Gehäuseteil II der Reinigungsvorrichtung 1 gemäß Figur 3 dient als Auffangraum 10 für die durch die Membran 3 hindurch geförderte Reinigungsflüssigkeit.

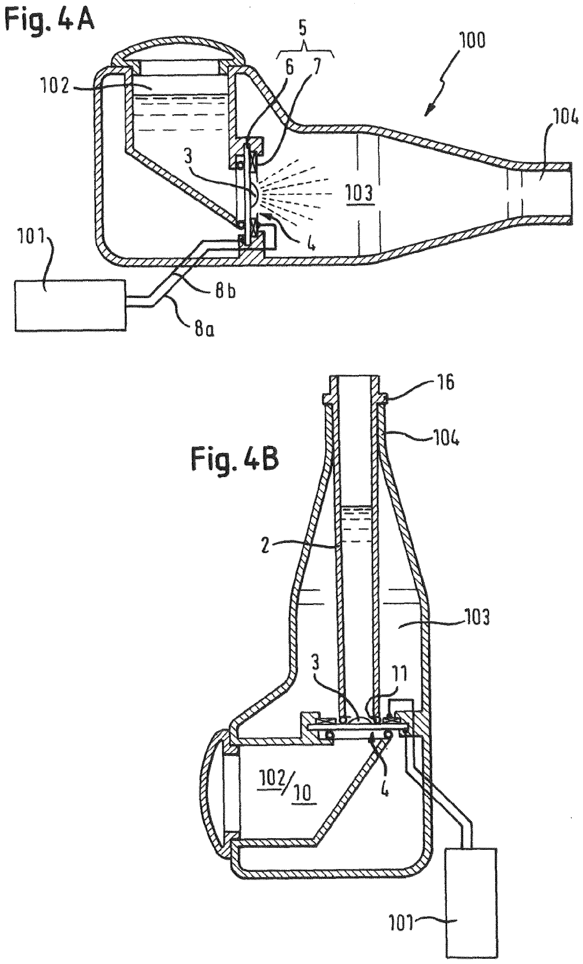

Figur 4A zeigt ein Inhalationstherapiegerät 100 mit einem Membranaerosolgenerator 4, der neben der Membran 3 einen Schwingungserzeuger 5 umfasst, der ein ringförmiges Substrat 6 und ein ringförmiges piezo-elektrisches Element 7 aufweist, die miteinander und mit der Membran 3 derart verbunden sind, dass die Membran 3 in Schwingungen versetzt wird, wenn der Schwingungserzeuger 5 durch ein elektrisches Ansteuersignal zu Schwingungen angeregt wird. Das elektrische Ansteuerungssignal wird über Zuleitungen 8a, 8b zugeführt, die an ein Steuergerät 101 des Inhalationstherapiegeräts 100 angeschlossen sind.

In ein Flüssigkeitsreservoir 102 des Inhalationstherapiegeräts 100 wird die zu vernebelnde Flüssigkeit eingefüllt und diese steht während des Verneblerbetriebs auf der Flüssigkeitsseite der Membran 3 an. Wenn von dem Steuergerät 101 ein Ansteuersignal dem Membranaerosolgenerator 4 zugeführt wird, wird die Membran 3 in Schwingungen versetzt, so dass die zu vernebelnde Flüssigkeit von der Flüssigkeitsseite auf die Aerosolseite der Membran 3 transportiert wird. Das Aerosol wird von dem Membranaerosolgenerator 4 in eine Mischkammer 103 abgegeben, aus der ein Patient das Aerosol über ein Mundstück 104 einatmet. Obwohl in Figur 4A einstückig dargestellt, ist das Mundstück 104 in vielen Fällen von der Mischkammer 103 abnehmbar.

Bei einem derart ausgestalteten Inhalationstherapiegerät 100 umfasst, wie in Figur 4B gezeigt ist, die Reinigungsvorrichtung gemäß der Erfindung eine Flüssigkeitszuführungseinrichtung 2, die in Form eines Rohres ausgebildet ist, das in das Mundstück 104 des Inhalationstherapiegeräts 100 eingeführt wird. Die Länge der Flüssigkeitszuführungseinrichtung 2 richtet sich nach der Größe des Mundstücks 104 und der Mischkammer 103 und ist so groß ausgelegt, dass eine offene Stirnseite der Flüssigkeitszuführungseinrichtung 2 die Membran 3 erreicht, so dass die an dieser Stirnseite vorgesehene Dichtung 11 an der Membran 3 oder dem Membranaerosolgenerator 4 anliegt. Sofern das Mundstück 104 von der Mischkammer 103 abnehmbar ist, kann die rohrartige Flüssigkeitszuführungseinrichtung 2 in ihren Abmessungen auch an die Mischkammer 103 angepasst werden.

Vorzugsweise besitzt die Flüssigkeitszuführungseinrichtung 2 einen Anschlag 16, der an dem Öffnungsrand des Mundstücks 104 bzw. der Mischkammer 103 des Inhalationstherapiegeräts anschlägt und dadurch verhindert, dass die Flüssigkeitszuführungseinrichtung 2 zu weit in das Inhalationstherapiegerät 100 eingeführt wird und dabei die Membran 3 oder den Membranaerosolgenerator 4 beschädigt. Daneben läuft das Rohr 2 vorzugsweise konisch zu, um das Einführen in das Mundstück 104 bzw. die Mischkammer 103 zu erleichtern und um an dem einen Ende einen stirnseitigen Querschnitt zu erreichen, der an die Membran 3 bzw. den Membranaerosolgenerator 4 angepasst ist, und an dem entgegengesetzten Ende einen stirnseitigen Querschnitt zu erreichen, der an die Öffnung des Mundstücks 104 bzw. der Mischkammer 103 angepasst ist.

In das Rohr 2, das bei dem vierten Ausführungsbeispiel die Flüssigkeitszuführungseinrichtung der erfindungsgemäßen Reinigungsvorrichtung bildet, wird die Reinigungsflüssigkeit eingefüllt, so dass diese auf der Aerosolseite der Membran 3 während des Reinigungsvorgangs ansteht. Die Membran 3 wird mit Hilfe des Steuergeräts 101 in Schwingungen versetzt, das auch zur Ansteuerung des Membranaerosolgenerators 4 für die Erzeugung eines Aerosols verwendet wird. Dadurch wird auf vorteilhafte Weise das Steuergerät 101 des Inhalationstherapiegeräts 100 auch für die Reinigung verwendet. Wenn ein Ansteuersignal von dem Steuergerät 101 dem Membranaerosolgenerator 4 zugeführt wird, wird die Membran 3 in Schwingungen versetzt, so dass die Reinigungsflüssigkeit, die mit Hilfe der Flüssigkeitszuführungseinrichtung 2 der Aerosolseite der Membran 3 zugeführt wird, durch die Membran 3 transportiert wird und im Flüssigkeitsreservoir 102 aufgefangen wird. Das Flüssigkeitsreservoir 102 des Inhalationstherapiegeräts 100 dient dabei als Auffangbehälter 10 für die Reinigungsflüssigkeit, die durch die Membran 3 hindurchgefördert wurde.

Um zu vermeiden, dass versehentlich die im Flüssigkeitsreservoir 102 aufgefangene Reinigungsflüssigkeit vernebelt und eingeatmet wird, sollte aber vorzugsweise bei der Reinigung der Deckel des Flüssigkeitsreservoirs 102 entfernt werden. Um die dann aus dem Flüssigkeitsreservoir 102 austretende Reinigungsflüssigkeit aufzufangen, muss ein separater Auffangbehälter 10 vorgesehen werden. Auf diese Weise wird auch bei dem in Figur 4B gezeigten Ausführungsbeispiel der allen Ausbildungen gemäß der Erfindung innewohnende weitere Vorteil sicher erreicht, dass keine Reinigungsflüssigkeit versehentlich vernebelt wird.

Bei dem zuletzt beschriebenen vierten Ausführungsbeispiel einer erfindungsgemäßen Reinigungsvorrichtung ist von besonderem Vorteil, dass die Reinigungsflüssigkeit der Aerosolseite der Membran 3 zugeführt wird, da auf diesem Weg verhindert wird, dass eine Reinigungsflüssigkeit, die in das Flüssigkeitsreservoir eingefüllt ist, versehentlich vernebelt und eingeatmet wird. Die Zuführung der Reinigungsflüssigkeit auf der Aerosolseite der Membran 3 verhindert, dass das Inhalationstherapiegerät 100 im Reinigungsmodus möglicherweise zum Vernebeln einer Reinigungsflüssigkeit verwendet wird.

Darüber hinaus ist das vierte Ausführungsbeispiel besonders vorteilhaft, weil das Steuergerät 101 des Inhalationstherapiegeräts 100 als erfindungsgemäße Schwingungsaktivierungseinrichtung 9 verwendet wird, um die Membran 3 in Schwingungen zu versetzen, damit die Reinigungsflüssigkeit von der Aerosolseite auf die Flüssigkeitsseite transportiert wird.

Auch bei den zuvor beschriebenen Ausführungsbeispielen kann das Steuergerät eines Inhalationstherapiegeräts verwendet werden, um das Ansteuersignal dem Membranaerosolgenerator 4 zuzuführen, damit die Membran 3 in Schwingungen versetzt wird, um den gewünschten Transport der Reinigungsflüssigkeit von der Aerosolseite auf die Flüssigkeitsseite der Membran 3 zu bewerkstelligen. Jedoch kann bei einer erfindungsgemäßen Reinigungsvorrichtung auch ein von dem Inhalationstherapiegerät unabhängiges Ansteuergerät als Schwingungsaktivierungseinrichtung verwendet werden, was die Möglichkeit bietet, andere Ansteuersignale, zum Beispiel in anderen Frequenzbereichen oder mit anderen Signalformen, bereitzustellen. Jedoch hat sich gezeigt, dass bei der Verwendung des Steuergeräts des Inhalationstherapiegeräts bei den Betriebsbedingungen, die auch für die Verneblung der Flüssigkeit zur Erzeugung eines Aerosols verwendet werden, gute Reinigungserfolge erzielt werden.

Mit der erfindungsgemäßen Kombination einer Reinigungsvorrichtung und einer Reinigungsflüssigkeit kann ein hochwirksames Verfahren zur Reinigung einer schwingungsfähigen Membran 3 eines Membranaerosolgenerators 4 eines Inhalationstherapiegeräts 100 durchgeführt werden. Dazu wird erfindungsgemäß der Membran 3 eine Reinigungsflüssigkeit zugeführt, wobei die Reinigungsflüssigkeit wenigstens ein Tensid enthält. Ferner wird erfindungsgemäß die Membran 3 derart in Schwingungen versetzt, dass die Reinigungsflüssigkeit durch die Öffnungen der Membran 3 gefördert wird.

Ausführungsbeispiele der erfindungsgemäßen Kombination umfassen die oben dargelegten Ausführungsbeispiele der Reinigungsvorrichtung zusammen mit einer Reinigungsflüssigkeit, die wenigstens ein Tensid enthält. Vorzugsweise enthält die Reinigungsflüssigkeit ferner wenigstens ein Enzym und/oder wenigstens eine Säure. Eine solche Kombination einer Reinigungsvorrichtung und einer Reinigungsflüssigkeit kann besonders vorteilhaft für die Durchführung des erfindungsgemäßen Verfahrens verwendet werden.

Insbesondere kann für die erfindungsgemäße Kombination und das erfindungsgemäße Verfahren eine Reinigungsflüssigkeit verwendet werden, die ein Tensid, ein Enzym und eine Säure enthält. In diesem Fall werden Verunreinigungen und Verstopfungen der Membran besonders effektiv entfernt, wie nachfolgend anhand von experimentellen Daten ausführlich dargelegt wird.

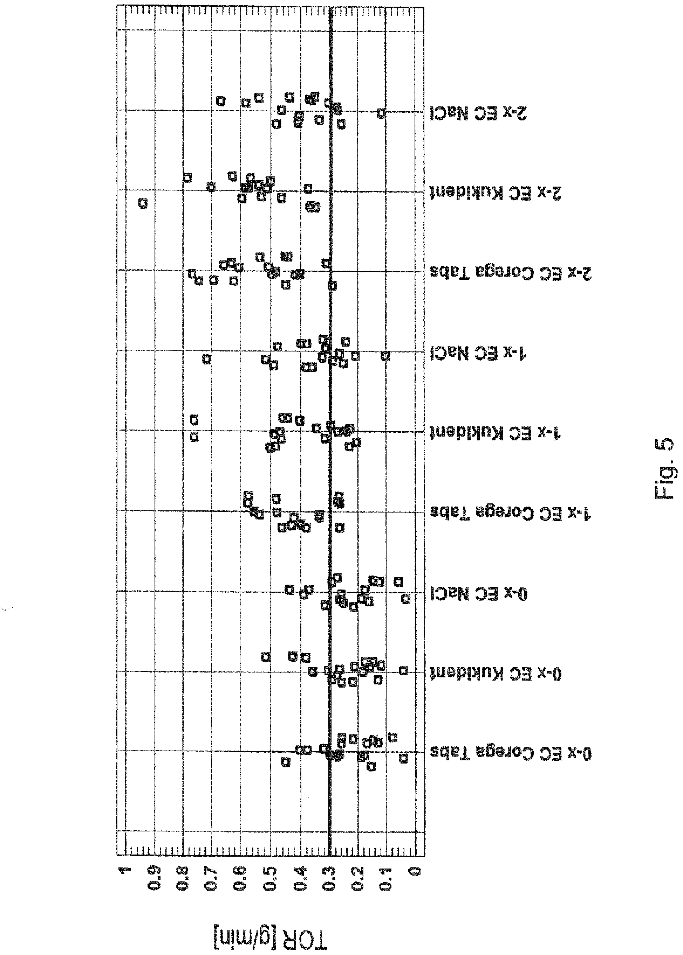

Figur 5 zeigt experimentelle Daten für die Vernebelungsraten ("Total Output Rate" [TOR]) schwingungsfähiger Membranen von Membranaerosolgeneratoren nach Anwendung unterschiedlicher Reinigungsverfahren. Die in Figur 5 gezeigten Daten wurden für drei Gruppen mit jeweils 18 Membranen erhalten, bei denen die Vernebelungsraten aufgrund von Verunreinigungen und Verstopfungen der Membranen zurückgegangen waren. Die ersten drei Datensätze auf der linken Seite der Figur 5 ("0-x EC Corega Tabs®; "0-x EC Kukident®"; "0-x EC NaCl") zeigen die Vernebelungsraten der Gruppen von Membranen im unbehandelten Zustand, also vor der Durchführung einer Reinigung. Wie der Figur 5 zu entnehmen ist, befinden sich die Vernebelungsraten dieser Membrangruppen im Wesentlichen im gleichen Bereich.

Die Vernebelungsraten (TOR) wurden gravimetrisch mit isotoner Kochsalzlösung (NaCl 0,9%) ermittelt. Alternativ könnte diese Wägung mit Medikamenten oder Prüfflüssigkeiten vermessen werden.

Jede der oben genannten drei Gruppen von Membranen wurde mit einem unterschiedlichen Reinigungsverfahren, d.h. unter Verwendung einer unterschiedlichen Reinigungsflüssigkeit, gereinigt. Insbesondere wurde allen Membranen eine Reinigungsflüssigkeit auf deren Aerosolseite zugeführt und wurden die Membranen derart in Schwingungen versetzt, dass die Reinigungsflüssigkeit durch die Öffnungen der Membranen auf die Flüssigkeitsseite der Membranen gefördert wurde. Als Reinigungsflüssigkeit wurde für die erste Gruppe von Membranen eine Corega-Tabs®-Lösung, für die zweite Gruppe von Membranen eine Kukident®-Lösung und für die dritte Gruppe von Membranen eine 0,9% NaCl-Lösung verwendet. Für die Corega-Tabs®-Lösung wurde eine Tablette des Zahnersatzreinigungsmittels Corega Tabs® Bio-Formel von GlaxoSmithKline in Leitungswasser aufgelöst. Für die Kukident-Lösung wurde eine Tablette des Zahnersatzreinigungsmittels Kukident von Reckitt Benckiser in Leitungswasser aufgelöst.

Die Vernebelungsraten der drei Membrangruppen nach Durchführung eines solchen Reinigungsdurchgangs sind in der Mitte der Figur 5 gezeigt ("1-x EC Corega Tabs®"; "1-x EC Kukident®"; "1-x EC NaCl"). Wie der Figur 5 zu entnehmen ist, weisen die mit der Corega-Tabs®-Lösung gereinigten Membranen und die mit der Kukident®-Lösung gereinigten Membranen eine höhere Vernebelungsrate als die mit der NaCl-Lösung gereinigten Membranen auf.

Anschließend wurde ein zweiter Reinigungsvorgang in der oben dargelegten Weise durchgeführt. Die nach diesem zweiten Reinigungsdurchgang erhaltenen Vernebelungsraten sind in Figur 5 auf der rechten Seite gezeigt ("2-x EC Corega Tabs®"; "2-x EC Kukident®"; "2-x EC NaCl"). Wie der Figur 5 zu entnehmen ist, wurde durch den zweiten Reinigungsdurchgang eine weitere Verbesserung der Vernebelungsraten für die mit der Corega®-Tabs-Lösung bzw. der Kukident®-Lösung gereinigten Membranen gegenüber den mit der NaCl-Lösung gereinigten Membranen erzielt.

Die durch die Verwendung der Corega®-Tabs-Lösung bzw. der Kukident®-Lösung gegenüber der Reinigung mit der NaCl-Lösung erzielte Steigerung der Reinigungseffizienz geht auch aus den nachfolgend gezeigten Tabellen 1 bis 3 hervor.

Tabelle 1 zeigt jeweils den Anteil der Membranen aus jeder der oben genannten drei Membrangruppen, der eine Vernebelungsrate von ≥ 300 mg/min (untere Spezifikation; durchgezogene horizontale Linie in Figur 5 bei TOR = 0,3 g/min) aufweist. Tabelle 2 zeigt jeweils den Anteil der Membranen aus jeder der oben genannten drei Membrangruppen, der eine Vernebelungsrate von ≥ 470 mg/min (mittlere Spezifikation) aufweist. Tabelle 3 zeigt die Mittelwerte und Abweichungen der in Fig. 5 gezeigten experimentellen Daten.

**Tabelle 1: Anteil Membranen mit TOR ≥ 300 mg/min**

| | NaCl-Lösung | Corega-Tabs | Kukident |
|---|---|---|---|
| Ausgangswerte | 22% | 22% | 28% |
| 1-mal Reinigung | 67% | 78% | 67% |
| 2-mal Reinigung | 78% | 94% | 100% |

**Tabelle 2: Anteil Membranen mit TOR ≥ 470 mg/min**

| | NaCl-Lösung | Corega-Tabs | Kukident |
|---|---|---|---|
| Ausgangswerte | 0% | 0% | 6% |
| 1-mal Reinigung | 22% | 33% | 28% |
| 2-mal Reinigung | 22% | 64% | 67% |

**Tabelle 3: Mittelwerte (Abweichungen) TOR in g/min**

| | NaCl-Lösung | Corega-Tabs | Kukident |
|---|---|---|---|
| Ausgangswerte | 0.226 (+/-0.110) | 0.232 (+/-0.110) | 0.246 (+/-0.119) |
| 1-mal Reinigung | 0.350 (+/-0.137) | 0.410 (+/-0.107) | 0.406 (+/-0.163) |
| 2-mal Reinigung | 0.388 (+/-0.129) | 0.525 (+/-0.138) | 0.538 (+/-0.157) |

Insbesondere geht aus Tabelle 1 hervor, dass bei der Reinigung mit der Kukident®-Lösung alle Membranen und bei der Reinigung mit der Corega®-Tabs-Lösung beinahe alle Membranen nach dem zweiten Reinigungsdurchgang eine Vernebelungsrate von ≥ 300 mg/min aufweisen. Überdies ist der Anteil der Membranen mit einer Vernebelungsrate ≥ 470 mg/min bei der Reinigung mit der Corega®-Tabs-Lösung oder der Kukident -Lösung nach dem zweiten Reinigungsdurchgang annähernd dreimal so hoch wie bei der Reinigung mit der NaCl-Lösung (siehe Tabelle 2). Die Steigerung der Effizienz des Reinigungsverfahrens durch die Verwendung der Corega®-Tabs-Lösung oder der Kukident®-Lösung gegenüber der Reinigung mit der NaCl-Lösung geht auch aus Tabelle 3 hervor.

Allgemein können bei der vorliegenden Erfindung die folgenden TOR-Spezifikationen für die Membran bestehen.

Die Spezifikation kann für Partikelgrößen (MMAD) von 2,0 *µ*m bis 4,0 *µ*m bei 100 mg/min liegen, bevorzugt bei 200 mg/min und bevorzugter bei 300 mg/min.

Die Spezifikation kann für MMAD von 3,0 *µ*m bis 5,0 *µ*m bei 300 mg/min liegen, bevorzugt bei 400 mg/min und bevorzugter bei 500 mg/min.

Die Spezifikation kann für MMAD von 3,5 *µ*m bis 6,5 *µ*m bei 600 mg/min liegen, bevorzugt bei 700 mg/min und bevorzugter bei 800 mg/min.

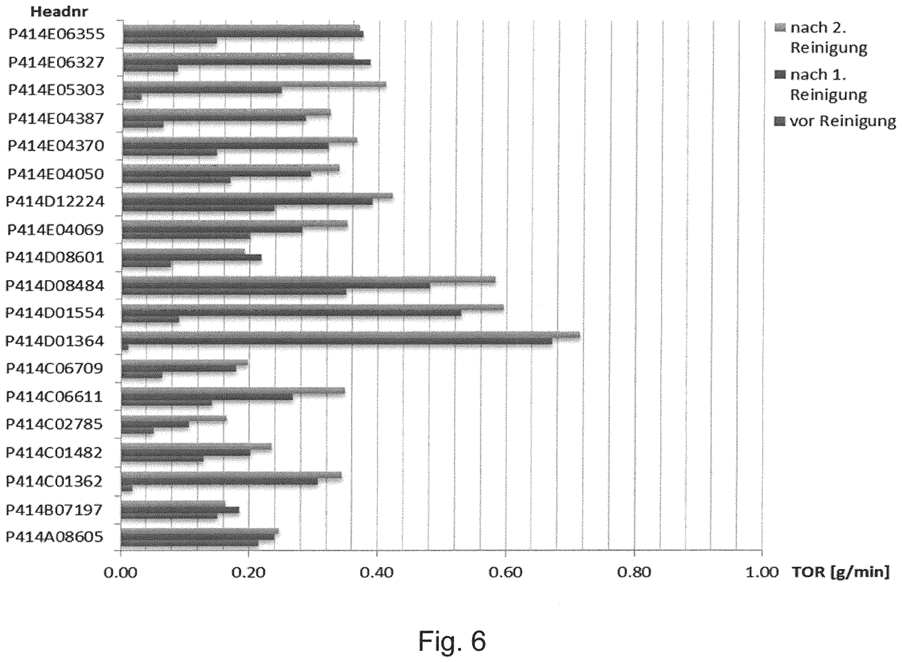

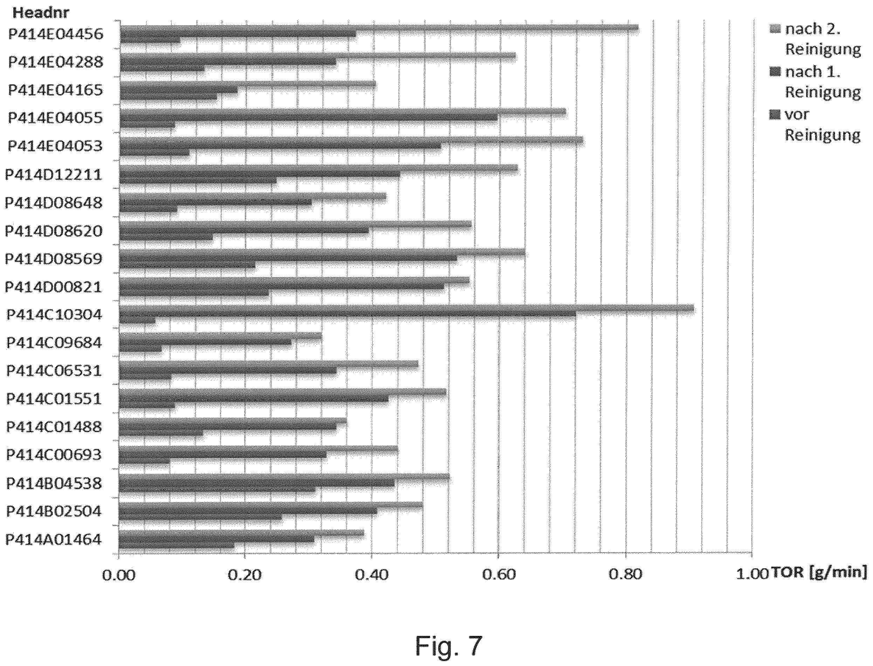

In einer weiteren experimentellen Untersuchung wurden zwei Gruppen von jeweils 19 Membranen, deren Vernebelungsraten aufgrund von Verunreinigungen und Verstopfungen zurückgegangen waren, einer Reinigung mit deionisiertem Wasser (erste Gruppe) bzw. einer Reinigung mit einer Corega®-Tabs-Lösung (zweite Gruppe) unterzogen. Die experimentellen Ergebnisse für die erste und die zweite Gruppe von Membranen sind in Figur 6 bzw. Figur 7 gezeigt. In diesen Figuren sind auf der Ordinate jeweils die Kennnummern ("Headnr") der Membranen aufgetragen, während die Abszisse die Vernebelungsraten ("TOR") in g/min zeigt.

In den Figuren 6 und 7 zeigt bei jeder Membran-Kennnummer der untere Balken die Vernebelungsrate vor der Reinigung, der mittlere Balken die Vernebelungsrate nach dem ersten Reinigungsdurchgang und der obere Balken die Vernebelungsrate nach dem zweiten Reinigungsdurchgang. Der erste und der zweite Reinigungsdurchgang wurden in der oben mit Bezug auf Figur 5 bereits dargelegten Weise durchgeführt.

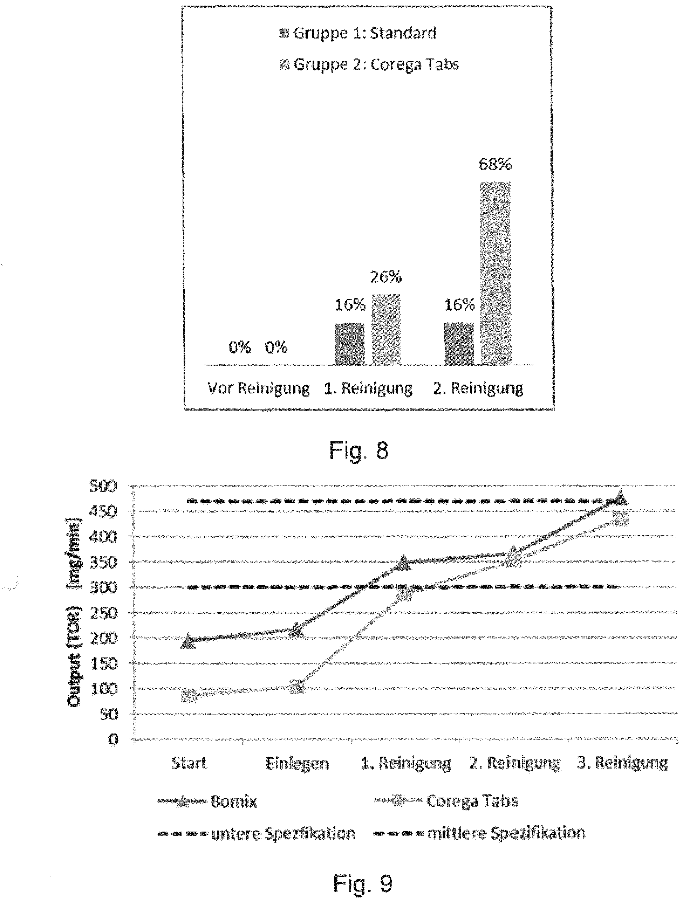

Wie aus einem Vergleich der Figur 6 mit der Figur 7 hervorgeht, wird die Effizienz des Reinigungsverfahrens durch die Verwendung der Corega®-Tabs-Lösung gegenüber der Reinigung mit deionisiertem Wasser erheblich gesteigert. Dies geht auch aus der Figur 8 hervor, die den prozentualen Anteil der Membranen der ersten bzw. zweiten Gruppe zeigt, die eine Vernebelungsrate von ≥ 470 mg/min aufweisen.

Wie der Figur 8 zu entnehmen ist, konnte durch die Verwendung der Corega®-Tabs-Lösung eine erheblich effizientere Reinigung der Membranen als durch die Verwendung von deionisiertem Wasser erzielt werden. Insbesondere weisen bei der Reinigung mit der Corega®-Tabs-Lösung 68% der Membranen nach dem zweiten Reinigungsdurchgang eine Vernebelungsrate von ≥ 470 mg/min, also eine Vernebelungsrate bei oder über der mittleren Spezifikation, auf.

Figur 9 zeigt experimentelle Daten für eine erste Membran (obere Kurve in Figur 9) und eine zweite Membran (untere Kurve in Figur 9) deren Vernebelungsraten aufgrund von Verunreinigungen und Verstopfungen zurückgegangen waren. Diese beiden Membranen wurden im Wesentlichen in der oben mit Bezug auf Figur 5 bereits erörterten Weise gereinigt, wobei die Membranen vor dem ersten Reinigungsdurchgang in der Reinigungsflüssigkeit eingelegt wurden und nach dem zweiten Reinigungsdurchgang ein dritter Reinigungsdurchgang durchgeführt wurde. Für die erste Membran wurde Bomix® Plus von der Paul Hartmann AG, das ein Desinfektionsmittel enthält, als Reinigungsflüssigkeit verwendet. Für die zweite Membran wurde eine wie oben beschriebene Corega®-Tabs-Lösung als Reinigungsflüssigkeit verwendet.

Wie der Figur 9 zu entnehmen ist, konnte sowohl durch die Reinigung mit Bomix® Plus als auch durch die Reinigung mit der Corega-Tabs®-Lösung eine erhebliche Steigerung der Vernebelungsrate auf einen Wert von ungefähr 450 mg/min nach dem dritten Reinigungsdurchgang erzielt werden. Überdies lagen die Vernebelungsraten der Membranen bereits nach dem ersten Reinigungsdurchgang über (erste Membran) bzw. nahe bei (zweite Membran) der unteren Spezifikation von 300 mg/min.

Die Erfindung ist nicht auf die beschriebenen Ausführungsformen beschränkt, sondern kann im Umfang der folgenden Patentansprüche modifiziert werden.

## Patentansprüche

1. Verfahren zur Reinigung einer schwingungsfähigen Membran (3) eines Membranaerosolgenerators (4) eines Inhalationstherapiegeräts (100), bei dem
der Membran (3) eine Reinigungsflüssigkeit zugeführt wird, und
die Membran (3) derart in Schwingungen versetzt wird, dass die Reinigungsflüssigkeit durch die Öffnungen der Membran (3) gefördert wird,
**dadurch gekennzeichnet, dass**
die Reinigungsflüssigkeit wenigstens ein Tensid und wenigstens ein Enzym enthält, und
das wenigstens eine Enzym ein Enzym zur Spaltung von Proteinen und/oder Peptiden ist.

2. Verfahren nach Anspruch 1, bei dem die Reinigungsflüssigkeit ferner wenigstens eine Säure enthält.

3. Verfahren nach Anspruch 2, bei dem die wenigstens eine Säure eine organische Säure, insbesondere Zitronensäure, ist.

4. Verfahren nach einem der vorhergehenden Ansprüche, bei dem
die Reinigungsflüssigkeit der Membran (3) auf der Aerosolseite zugeführt wird, und
die Membran (3) derart in Schwingungen versetzt wird, dass die Reinigungsflüssigkeit durch die Öffnungen der Membran (3) auf die Flüssigkeitsseite der Membran (3) gefördert wird.

5. Verfahren nach einem der vorhergehenden Ansprüche, bei dem die Reinigungsflüssigkeit ferner wenigstens ein Desinfektionsmittel und/oder wenigstens ein Wasserenthärtungsmittel und/oder wenigstens ein Lösungsmittel enthält.

6. Verfahren nach einem der vorhergehenden Ansprüche, bei dem die Reinigungsflüssigkeit ferner wenigstens einen Hilfsstoff, insbesondere einen Puffer, enthält.

7. Verfahren nach einem der vorhergehenden Ansprüche, bei dem die Membran (3) auf dieselbe Art in Schwingungen versetzt wird, in der die Membran (3) im Verneblerbetrieb in Schwingungen versetzt wird.

8. Kombination einer Reinigungsvorrichtung (1) und einer Reinigungsflüssigkeit, wobei die Reinigungsvorrichtung (1) für die Reinigung einer schwingungsfähigen Membran (3) eines Membranaerosolgenerators (4) eines Inhalationstherapiegeräts (100) ausgelegt ist und umfasst:
- eine Flüssigkeitszuführungseinrichtung (2), die für die Zuführung der Reinigungsflüssigkeit zu der Membran (3) ausgelegt ist, und
- eine Schwingungsaktivierungseinrichtung (9), die für die Aktivierung von Membranschwingungen, so dass die Reinigungsflüssigkeit durch die Öffnungen der Membran (3) gefördert wird, ausgelegt ist,
**dadurch gekennzeichnet, dass**
die Reinigungsflüssigkeit wenigstens ein Tensid und wenigstens ein Enzym enthält, und
das wenigstens eine Enzym ein Enzym zur Spaltung von Proteinen und/oder Peptiden ist.

9. Kombination nach Anspruch 8, bei der die Reinigungsflüssigkeit ferner wenigstens eine Säure enthält.

10. Kombination nach Anspruch 8 oder 9, bei der
die Flüssigkeitszuführungseinrichtung (2) für die Zuführung der Reinigungsflüssigkeit auf die Aerosolseite der Membran (3) ausgelegt ist, und
die Schwingungsaktivierungseinrichtung (9) für die Aktivierung von Membranschwingungen, so dass die Reinigungsflüssigkeit durch die Öffnungen der Membran (3) auf die Flüssigkeitsseite der Membran (3) gefördert wird, ausgelegt ist.

11. Kombination nach einem der Ansprüche 8 bis 10, die für die Durchführung des Verfahrens nach einem der Ansprüche 1 bis 7 ausgelegt ist.

12. Kombination nach einem der Ansprüche 8 bis 11, bei der die Flüssigkeitszuführungseinrichtung (2) ein Hohlzylinder ist, der mit einer Stirnseite an der Membran (3) oder dem Membranaerosolgenerator (4) derart angeordnet ist, dass eine in den Hohlzylinder eingefüllte Reinigungsflüssigkeit an der Membran (3) ansteht.

13. Kombination nach Anspruch 12, bei der der Hohlzylinder als längliches Rohr ausgebildet ist, das für das Einsetzen in ein Mundstück (104) oder in eine Mischkammer (103) des Inhalationstherapiegeräts (100) ausgelegt ist.

14. Kombination nach einem der Ansprüche 8 bis 11, bei der die Flüssigkeitszuführungseinrichtung (2) trichterförmig ist und mit einer Öffnung an der Membran (3) oder dem Membranaerosolgenerator (4) derart angeordnet ist, dass eine in die trichterförmige Flüssigkeitszuführungseinrichtung (2) eingefüllte Reinigungsflüssigkeit an der Membran (3) ansteht.

## Claims

1. Method for cleaning a vibratory membrane (3) of a membrane aerosol generator (4) of an inhalation therapy device (100), wherein
a cleaning liquid is fed to the membrane (3), and
the membrane (3) is caused to vibrate in such a way that the cleaning liquid is transported through the openings in the membrane (3),
**characterised in that**
the cleaning liquid contains at least one surfactant and at least one enzyme, and
the at least one enzyme is an enzyme for splitting proteins and/or peptides.

2. Method according to claim 1, wherein the cleaning liquid further contains at least one acid.

3. Method according to claim 2, wherein the at least one acid is an organic acid, in particular citric acid.

4. Method according to one of the preceding claims, wherein
the cleaning liquid is fed to the membrane (3) on the aerosol side, and
the membrane (3) is caused to vibrate in such a way that the cleaning liquid is transported through the openings in the membrane (3) on the liquid side of the membrane (3).

5. Method according to one of the preceding claims, wherein the cleaning liquid further contains at least one disinfectant and/or at least one water-softening agent and/or at least one solvent.

6. Method according to one of the preceding claims, wherein the cleaning liquid further contains at least one excipient, in particular a buffer.

7. Method according to one of the preceding claims, wherein the membrane (3) is caused to vibrate in the same manner in which the membrane (3) is caused to vibrate in nebuliser operation.

8. Combination of a cleaning device (1) and a cleaning liquid, wherein the cleaning device (1) is designed to clean a vibratory membrane (3) of a membrane aerosol generator (4) of an inhalation therapy device (100) and comprises:
- a liquid feed device (2) which is designed to feed the cleaning liquid to the membrane (3), and
- a vibration activation device (9) which is designed to activate membrane vibrations so that the cleaning liquid is transported through the openings in the membrane (3), **characterised in that**
the cleaning liquid contains at least one surfactant and at least one enzyme, and
the at least one enzyme is an enzyme for splitting proteins and/or peptides.

9. Combination according to claim 8, wherein the cleaning liquid further contains at least one acid.

10. Combination according to claim 8 or 9, wherein
the liquid feed device (2) is designed to feed the cleaning liquid on the aerosol side of the membrane (3), and
the vibration activation device (9) is designed to activate membrane vibrations so that the cleaning liquid is transported through the openings in the membrane (3) on the liquid side of the membrane (3).

11. Combination according to one of the claims 8 to 10 which is designed to carry out the method according to one of the claims 1 to 7.

12. Combination according to one of the claims 8 to 11, wherein the liquid feed device (2) is a hollow cylinder which is arranged with an end face on the membrane (3) or the membrane aerosol generator (4) in such a way that a cleaning liquid filled into the hollow cylinder lies against the membrane (3).

13. Combination according to claim 12, wherein the hollow cylinder is designed as an elongated tube which is designed to be fitted into a mouthpiece (104) or into a mixing chamber (103) of the inhalation therapy device (100) .

14. Combination according to one of the claims 8 to 11, wherein the liquid feed device (2) is funnel-formed and is arranged with an opening on the membrane (3) or the membrane aerosol generator (4) in such a way that a cleaning liquid filled into the funnel-formed liquid feed device (2) lies against the membrane (3).

## Revendications

1. Procédé de nettoyage d'une membrane (3) oscillante d'un générateur d'aérosol à membrane (4) d'un appareil de thérapie par inhalation (100), dans lequel
la membrane (3) est alimentée en liquide de nettoyage et
la membrane (3) est mise en oscillation de sorte que le liquide de nettoyage est propulsé à travers les ouvertures de la membrane (3),
**caractérisé en ce que**
le liquide de nettoyage contient au moins un tensioactif et au moins une enzyme, et
**en ce qu'**au moins une enzyme est une enzyme destinée au clivage des protéines et/ou des peptides.

2. Procédé selon la revendication 1, dans lequel le liquide de nettoyage contient en outre au moins un acide.

3. Procédé selon la revendication 2, dans lequel au moins un acide est un acide organique, en particulier de l'acide citrique.

4. Procédé selon l'une quelconque des revendications précédentes, dans lequel
le liquide de nettoyage de la membrane (3) est alimenté du côté de l'aérosol, et
la membrane (3) est mise en oscillation, de sorte que le liquide de nettoyage est propulsé à travers les ouvertures de la membrane (3) du côté du liquide de la membrane (3).

5. Procédé selon l'une quelconque des revendications précédentes, dans lequel le liquide de nettoyage contient en outre au moins un désinfectant et/ou au moins un agent d'adoucisseur d'eau et/ou au moins un solvant.

6. Procédé selon l'une quelconque des revendications précédentes, dans lequel le liquide de nettoyage contient en outre au moins un adjuvant, en particulier un tampon.

7. Procédé selon l'une quelconque des revendications précédentes, dans lequel la membrane (3) est mise en oscillation de la même manière, dans laquelle la membrane (3) est mise en oscillation dans un nébuliseur.

8. Combinaison d'un dispositif de nettoyage (1) et d'un liquide de nettoyage, dans laquelle le dispositif de nettoyage (1) est conçu pour le nettoyage d'une membrane (3) oscillante d'un générateur d'aérosol à membrane (4) d'un appareil de thérapie par inhalation (100) et comprend :
- un dispositif d'alimentation en liquide (2), qui est conçu pour l'alimentation de la membrane (3) en liquide de nettoyage, et
- un dispositif d'activation des oscillations (9), qui est conçu pour l'activation des oscillations de la membrane, de sorte que le liquide de nettoyage est propulsé à travers les ouvertures de la membrane (3),
**caractérisé en ce que**,
le liquide de nettoyage contient au moins un tensioactif et au moins une enzyme, et
**en ce qu'**au moins une enzyme est une enzyme destinée au clivage des protéines et/ou des peptides.

9. Combinaison selon la revendication 8, dans laquelle le liquide de nettoyage contient en outre au moins un acide.

10. Combinaison selon la revendication 8 ou 9, dans laquelle
le dispositif d'alimentation en liquide (2) est conçu pour l'alimentation de la membrane (3) en liquide de nettoyage du côté de l'aérosol, et
le dispositif d'activation des oscillations (9) est conçu pour l'activation des oscillations de la membrane, de sorte que le liquide de nettoyage est propulsé à travers les ouvertures de la membrane (3) du côté du liquide de la membrane (3).

11. Combinaison selon l'une quelconque des revendications 8 à 10, qui est conçue pour la réalisation du procédé selon l'une quelconque des revendications 1 à 7.

12. Combinaison selon l'une quelconque des revendications 8 à 11, dans laquelle le dispositif d'alimentation en liquide (2) est un cylindre creux, qui est disposé avec un côté avant au niveau de la membrane (3) ou du générateur d'aérosol à membrane (4) de sorte qu'un liquide de nettoyage introduit dans le cylindre creux est attenant à la membrane (3).

13. Combinaison selon la revendication 12, dans laquelle le cylindre creux est construit sous forme de tube allongé, qui est conçu pour l'insertion dans un embout buccal (104) ou dans une chambre de mélange (103) de l'appareil de thérapie par inhalation (100).

14. Combinaison selon l'une quelconque des revendications 8 à 11, dans laquelle le dispositif d'alimentation en liquide (2) est en forme d'entonnoir et disposé avec une ouverture au niveau de la membrane (3) ou du générateur d'aérosol à membrane (4) de sorte qu'un liquide de nettoyage introduit dans le dispositif d'alimentation en liquide (2) en forme d'entonnoir est attenant à la membrane (3).
